# EUROPEAN PATENT SPECIFICATION

(11) **EP 0 489 108 B1**
(45) Date of publication and mention of the grant of the patent: **13.03.1996**
(21) Application number: 90913638.4
(22) Date of filing: 15.08.1990
(51) Int. Cl.: A61K 31/445, A61K 31/475

(54) **Use of spiperone derivatives for immunosuppression**
Verwendung von Spiperone-derivate zur Immunosuppression
Utilisation des derivatives de spiperone pour immunosuppresion

(30) Priority: 21.08.1989 US 396523; 16.03.1990 US 494744
(43) Date of publication of application: 10.06.1992
(73) Proprietor: Beth Israel Hospital Association, Boston, Massachusetts 02215 (US)
(72) Inventor: SHARPE, Richard, J., Newtonville, MA 02160 (US); ARNDT, Kenneth, A., Newton Centre, MA 02159 (US); GALLI, Stephen, J., Winchester, MA 01890 (US)
(74) Representative: Bassett, Richard Simon
(86) International application number: US9004637
(87) International publication number: WO9102527

(56) References cited:
- EP-A- 0 268 309
- US-A- 2 788 309
- US-A- 2 854 380
- ADVANCES IN THERAPY, vol. 5, no. 4, July/August 1988, pages 143-152; H. TYTGAT et al.: "Topical ketanserin in the treatment of decubitus ulcers: A double-blind study with 2% ketanserin ointment against placebo"
- BLOZVSKI et al., Arch intern. Pharmacodynamie 123, 58-66 1959; CHEMICAL ABSTRACTS, Vol. 54, 1960, Abstract 21504h-i
- LOFFMAN et al.; America Heart Journal, 74(2) 229-34, 1967, CHEMICAL ABSTRACTS Vol. 67, 1967, Abstract 81019W

## Description

### Background of the Invention

The field of the invention is topical treatment of cutaneous, ocular, and mucosal hypersensitivity, inflammation, and hyperproliferative conditions.

Cutaneous, ocular, and mucosal inflammation, the development of changes in vascular tone and permeability and the associated infiltration of the skin, ocular, or mucosal tissues by leukocytes in response to endogenous or exogenous stimuli, probably evolved as a defense mechanism against infectious agents. However, even in healthy adults, cutaneous, ocular, and mucosal inflammation can occur in response to certain plant resins, such as those of poison ivy, and other commonly encountered agents in the environment. In individuals sensitized to these agents, a severe contact reaction can result upon exposure, with significant associated morbidity. Inflammation also occurs in association with reactions to physical agents such as sunlight and in association with thermal, electrical, or chemical burns. Severe or repeated inflammatory reactions can be followed by significant chronic changes, such as scarring of affected tissues. In some anatomical sites, such as the eye, these chronic changes can have serious long-term consequences, including diminished vision or actual blindness.

It is now widely recognized that much cutaneous, ocular, and mucosal inflammation is pathological in nature. For example, in atopic dermatitis and eczema in general, leukocyte (particularly mononuclear cells, lymphocytes, neutrophils, and eosinophils) infiltration into the skin is a general phenomenon and is important in the pathogenesis of these diseases. Similarly, psoriasis, a common cutaneous disease associated with a hyperproliferating epidermis, also has an inflammatory component. It is now believed that cells found in the normal and abnormal skin, eye, or mucosal membrane secrete cytokines which are important in recruiting inflammatory cells into these sites and in inducing chronic changes such as scarring.

In addition to contact dermatitis, atopic dermatitis, and eczema, other conditions involving pathogenic cutaneous, ocular, and mucosal inflammation include, but are not limited to psoriasis, icthyosis, acne vulgaris, alopecia areata, male and female pattern alopecia, arthropod bite reactions, pyoderma gangrenosa, lichen planus, cutaneous lupus erythematosus, scleroderma, mycosis fungoides, drug eruptions, and burns. These conditions may result in any one or more of the following symptoms or signs: itching, swelling, reddening, blisters, crusting, pain, scaling, cracking, hair loss, scarring, or oozing of fluid involving the skin, eye, or mucosal membranes.

The potential therapeutic benefits of controlling pathological cutaneous, ocular, or mucosal hypersensitivity, inflammation, hyperproliferation, or scarring has led to a search for therapeutic agents which are both safe and effective. Several substances are known to have the capability of inhibiting cutaneous leukocyte responses or hyperproliferative responses. Corticosteroids when administered systemically are effective in this regard but are associated with significant and potentially dangerous side effects. Topically applied corticosteroids have some efficacy in treating these conditions, but are only partially effective in many instances, and have their own significant side effects. Cyclosporine A when given systemically is also partially efficacious, but of little or no utility when applied topically. Cyclosporine A is also associated with the real potential of serious toxicity to several major organ systems. Other agents with partial utility for treating some of the above conditions include, psoralen plus ultraviolet A (PUVA), dapsone, and anti-malarials, but the risk-to-benefit ratios for these agents is unfavorable for most of these conditions.

There is a significant and very long-standing need to identify agents which can be applied topically to prevent or suppress (i.e. "treat") cutaneous, ocular, or mucosal hypersensitivity reactions, inflammation, hyperproliferation, or scarring, and which have favorable benefit to risk ratios. Optimally such agents should primarily act locally, and systemic absorption should not result in blood levels high enough to cause significant systemic toxicity.

### Summary of the Invention

The invention provides the use of a compound having the structure 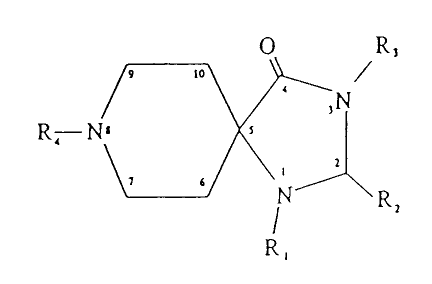
wherein
- R₁ =: H, CH₃-, C₆H₅-, cyclohexyl, 4-(OCH₃)C₆H₄-, 3-(CH₃)C₆H₄-, 4-(CH₃)C₆H₄-, 4-X-C₆H₄-, (CH₃)₂CH-, CH₃(CH₂)₃-, (CH₃)₂CHCH₂-, CH₃CH₂CH(CH₃)-, or (CH₃)₃C-;
- R₂ =: H or CH₃;
- R₃ =: H, CH₃, CH₃CH₂-, CH₃CH₂CH₂-, (CH₃)₂CH-, or CN(CH₂)₂-;
- R₄ =: H, C₆H₅CH(CH₂CH₃)CH₂-, C₆H₅CH(CH₃)(CH₂)₂-, C₆H₅CH₂CH(CH₃)CH₂-, C₆H₅CH₂CH₂CH(CH₃)-, C₆H₅CH(CH₃)(CH₂)₃-, 4-CH₃C₆H₄CH(CH₃)(CH₂)₃-, 4-(CH₃O)C₆H₄CH(CH₃)(CH₂)₃, 4-X-C₆H₄CH(CH₃)CH₂-, 4-X-C₆H₄CH(CH₂CH₃)CH₂-, 4-X-C₆H₄CH(CH₃)(CH₂)₂-, 4-X-C₆H₄-CH(CH₃)(CH₂)₃- C₆H₅CH(OCH₃)(CH₂)₂-, 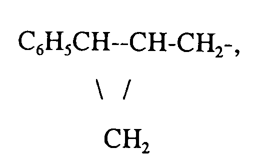 C₆H₅CO(CH₂)₃-, C₆H₅CO(CH₂)₄-, 4-(CH₃)C₆H₄CO(CH₂)₃-, 4-(CH₃O)C₆H₄CO(CH₂)₃-, 4-X-C₆H₄CO(CH₂)₃-,4-X-C₆H₄CO(CH₂)₃-, 2-thienyl-CO(CH₂)₃-, or 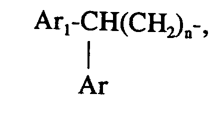
wherein n = 3 or 4; X = F, Cl, or Br; and each of Ar and Ar₁ is, independently, H, C₆H₅-, 4-(CH₃)C₆H₄-, 4-(CH₃O)C₆H₄-, 4-X-C₆H₄-, 3-(CX₃)C₆H₄-, 2-thienyl, or 4-X-C₆H₄CH₂-in the manufacture of a medicament for immunosuppression.

The composition contains a therapeutic amount of a rauwolfia alkaloid (an alkaloid derived from the Rauwolfia genus of plants) such as reserpine (a serotonin antagonist) or related compounds such as other serotonin antagonists which include but are not limited to ketanserin, cyproheptadine, spiperone, methysergide, LY 53857 (Lum and Piercey, Eur. J. Pharmacol. 149:9-15, 1988), ritanserin, ICI 169-369 (Goldstein et al., J. Pharmacol. Exp. Ther. 249:673-680, 1989), risperidone, pipamperone, trazodone, cinanserine, mianserin, and LY 281067 (Foreman et al., Life Sciences 45:1263-1270, 1989).

It is an advantage of the present invention that the use of the compositions provides topical treatment of cutaneous, ocular, or mucosal hypersensitivity reactions, inflammation, hyperproliferation or scarring, in a fashion that limits significant systemic effects.

The subject invention concerns use of compositions for the inhibition of cutaneous, ocular or mucosal hypersensitivity reactions, inflammation, hyperproliferation, or scarring. The preferred composition described herein comprises a rauwolfia alkaloid or serotonin antagonist, such as reserpine, in vehicles suitable for topical application and cutaneous, ocular or mucosal absorption. In tests conducted in accordance with the present invention, many such compositions have been shown to be effective in inhibiting cutaneous contact hypersensitivity reactions at the site of topical application, at doses that produce little or no inhibition of this response at a site distant from the site of topical application. In addition, topical application of a composition including spiperone induces inhibition of cutaneous contact hypersensitivity or inflammatory reactions at both the site of application and at a distant site, indicating that this particular serotonin antagonist may have significant systemic activity when applied to a cutaneous surface.

In accordance with the present invention, it has been discovered that the properties of reserpine, spiperone, and other related compounds, such as other serotonin antagonists, make them useful as topical agents in treating contact dermatitis, atopic dermatitis, eczematous dermatitis, drug eruptions, lichen planus, icthyosis pyoderma gangrenosa, psoriasis, alopecia areata, male and female pattern alopecia, cutaneous lupus erythematosus, scleroderma, inflammatory acne, arthropod bite reactions, aphthous ulcer, conjunctivitis, iritis, keratoconjunctivitis, vaginitis, proctitis, chemical burns, thermal burns, and photosensitivity conditions including sunburn. The compounds may also be useful in reducing the infiltration of skin by malignant leukocytes in diseases such as mycosis fungoides.

In its broadest overall aspect the composition is simply a rauwolfia alkaloid or a serotonin antagonist, of the type described above, dissolved or suspended in a suitable carrier. The composition is applied directly onto the affected area of the skin, eye, or mucosal membrane.

### Brief Description of the Drawings

Fig. 1 is a graph which shows the effects of topical reserpine on cutaneous contact hypersensitivity reactions (inflammation). The x axis represents time points 0, 24 and 48 hours after challenging both ears of mice with oxazolone, and the y axis is measurement of total ear thickness.

Fig. 2 is a bar graph illustrating the effect of topical administration of spiperone on ear swelling, expressed as changes (Δ) in ear thickness (post challenge value minus baseline prechallenge value) in mm x 10⁻², associated with oxazolone-induced contact hypersensitivity.

Fig. 3 is a bar graph illustrating the effect of topical administration of spiperone on numbers of infiltrating inflammatory cells, expressed as number of inflammatory cells per mm² of dermis, associated with oxazolone-induced contact hypersensitivity.

Fig. 4 is a bar graph illustrating the effect of topical administration of spiperone on ear swelling (expressed as in Fig. 2) associated with DNFB-induced contact hypersensitivity.

Fig. 5 is a bar graph illustrating the effect of topical administration of spiperone on numbers of infiltrating inflammatory cells (expressed as in Fig. 3) associated with DNFB-induced contact hypersensitivity.

Fig. 6 is a bar graph illustrating the effect of topical administration of spiperone on numbers of infiltrating inflammatory cells (expressed as in Fig. 3) associated with IL-1-induced inflammation.

### Detailed Description of the Invention

The subject invention is based on the discovery that cutaneous, ocular, or mucosal hypersensitivity reactions, inflammation, epithelial hyperproliferation, or scarring, can be treated by topical formulations of reserpine and/or spiperone (serotonin antagonists), and related compounds. Moreover, for many of these compounds, this effect can be directed to the site of application and immediate surrounding area without a significant similar systemic effect.

The conditions that the subject invention is therapeutically beneficial in treating include cutaneous hypersensitivity/inflammatory conditions such as contact dermatitis, atopic dermatitis, eczematous dermatitis, lichen planus, drug eruptions, cutaneous lupus erythematosus, scleroderma, pyoderma gangrenosa, alopecia areata, male and female pattern alopecia, inflammatory acne, arthropod bite reactions, burns, and photosensitivity conditions, including sunburn; cutaneous epidermal hyperproliferative conditions such as psoriasis and icthyosis; and mucosal hypersensitivity/inflammatory conditions such as lichen planus, aphthous ulcers, vaginitis, proctitis, conjunctivitis, iritis and keratoconjunctivitis. Additionally, suppression of a chronic inflammatory condition can prevent or lessen scar formation caused by the inflammation.

The subject invention pertains not only to reserpine, a rauwolfia alkaloid with serotonin antagonist properties, and spiperone, another serotonin antagonist, but also to other related compounds which have similar biologic activity with respect to the control of inflammation, function, migration, and proliferation of cutaneous and mucosal cells. Other such compounds may include, but are not limited to other rauwolfia alkaloids and serotonin antagonists such as ketanserin, cyproheptadine, methysergide, LY 53857, ritanserin, ICI 169-369, risperidone, pipamperone, trazodone, cinanserine, mianserin, LY 281067, and analogues and derivatives thereof.

Reserpine and some of the above related compounds are presently used for treating hypertension and psychiatric disorders. Reserpine, which depletes stores of serotonin (5-hydroxy-tryptamine) in many organs, has been shown to be effective in suppressing hypersensitivity responses in rodents when administered in high doses systemically. The physical properties of reserpine, spiperone, and the other related compounds are well documented. By applying these agents topically, therapeutic local concentrations are attainable without, for many of the compounds, the associated systemic side effects. Spiperone, however, under certain conditions (e.g. high doses) has been found to act to suppress hypersensitivity and inflammation both systematically and locally, when applied as a topical preparation to cutaneous surfaces.

Reserpine that is used in the present invention can be generally represented by the formula: 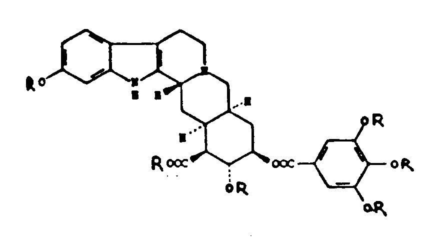
wherein each R = a hydrogen or alkyl of 1 to 6 carbon atoms. The form of reserpine utilized in Example 1 has the formula: 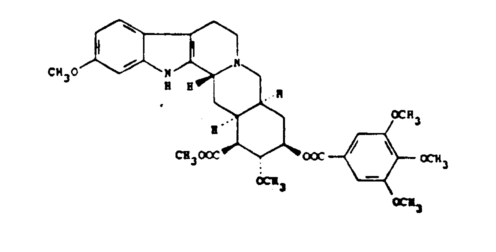
The term "spiperone" herein denotes all of the molecules which are effective in the method of the invention and which are the subject of the following US-A-3,155,669; US-A-3,155,670; US-A-3,161,644; and US-A-3,238,216. Methods for the synthesis of each such compound are disclosed in said four patents.

More particularly, forms of spiperone having the following formulae may be employed in the method of the invention: 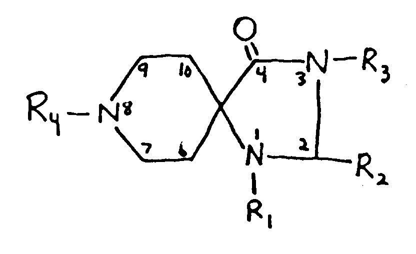
wherein
- R₁ =: H, CH₃-, C₆H₅-, cyclohexyl, 4-(OCH₃)C₆H₄-, 3-(CH₃)C₆H₄-, 4-(CH₃)C₆H₄-, 4-X-C₆H₄-, (CH₃)₂CH-, CH₃(CH₂)₃-, (CH₃)₂CHCH₂-, CH₃CH₂CH(CH₃)-, or (CH₃)₃C-;
- R₂ =: H or CH₃;
- R₃ =: H, CH₃, CH₃CH₂-, CH₃CH₂CH₂-, (CH₃)₂CH-, or CN(CH₂)₂-;
- R₄ =: H, C₆H₅CH(CH₂CH₃)CH₂-, C₆H₅CH(CH₃)(CH₂)₂-, C₆H₅CH₂CH(CH₃)CH₂-, C₆H₅CH₂CH₂CH(CH₃)-, C₆H₅CH(CH₃)(CH₂)₃-, 4-CH₃C₆H₄CH(CH₃)(CH₂)₃-, 4-(CH₃O)C₆H₄CH(CH₃)(CH₂)₃, 4-X-C₆H₄CH(CH₃)CH₂-, 4-X-C₆H₄CH(CH₂CH₃)CH₂-, 4-X-C₆H₄CH(CH₃)(CH₂)₂-, 4-X-C₆H₄-CH(CH₃)(CH₂)₃-, C₆H₅CH(OCH₃)(CH₂)₂-, 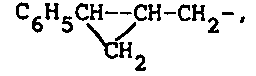 C₆H₅CO(CH₂)₃-, C₆H₅CO(CH₂)₄-, 4-(CH₃)C₆H₄CO(CH₂)₃-, 4-(CH₃O)C₆H₄CO(CH₂)₃-, 4-X-C₆H₄CO(CH₂)₃-, 4-X-C₆H₄CO(CH₂)₃-, 2-thienyl-CO(CH₂)₃-, or 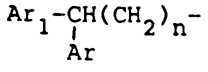
wherein n = 3 or 4; X= F, Cl, or Br; and each of Ar and Ar₁ is, independently, H, C₆H₅-, 4-(CH₃)C₆H₄-, 4-(CH₃O)C₆H₄-, 4-X-C₆H₄ -, 3-(CX₃)C₆H₄-, 2-thienyl, or 4-X-C₆H₄CH₂-.

Those forms of spiperone which are particularly useful in the method of the invention include those in which R₁ is C₆H₅-, 4-(X)-C₆H₄- or 4-(CH₃)C₆H₄-; R₂ is H or CH₃; R₃ is H, CH₃, or CH₃CH₂-; and R₄ is 4-X-C₆H₄CO(CH₂)₃- or 2-thienyl-CO(CH₂)₃-; and especially those in which R₁ is C₆H₅-, 4-(Br)-C₆H₄-, or 4-(Cl)-C₆H₄-; R₂ is H or CH₃; R₃ is H, CH₃, or CH₃CH₂-; and R₄ is 4-X-C₆H₄CO(CH₂)₃- or 2-thienyl-CO(CH₂)₃-.

The particular serotonin antagonist used in Examples 2-4 is 8-[3-(ρ-fluorobenzoyl)propyl]-1-phenyl-1,3,8-triazaspiro[4.5]decan-4-one. Herein termed "spiperone (1)". this compound has the following structure: 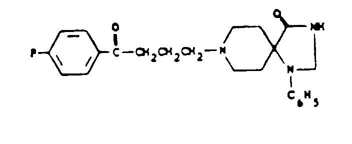
The potential utility of any one of the above-described forms of spiperone as a topical immunosuppressant and/or anti-inflammatory agent can be conveniently determined by synthesizing the compound and testing it in one or more of the biological assays described in Examples 2-4.

Furthermore, the reserpine and spiperone of the subject invention, and the other related compounds showing similar biologic activity, can be modified in order to enhance their usefulness as pharmaceutical compositions. For example, it is well known in the art that various modifications, such as alteration of charge, can alter water and lipid solubility and thus alter the potential for percutaneous absorption and for crossing the blood-brain barrier. The vehicle can be similarly modified to enhance cutaneous absorption, enhance the reservoir effect, and minimize potential irritancy or neuropharmacological effects of the composition. Additionally, the topical formulation can be occluded to further enhance absorption. Preservatives, stabilizers, emulsifiers, emulsion stabilizers, antioxidants, chelating agents, solvents, thickening agents, emollients, and humectants may be necessary or useful as part of the topical formulation (Arndt, K.A., P.V. Mendenhall [1987]) The Pharmacology of Topical Therapy. In Dermatology in General Medicine. T.B. Fitzpatrick, A.Z. Eisen, K. Wolff, I.M. Freedberg and K.F. Austen, eds., 3d ed., McGraw Hill, Inc., New York, pp.2532- 2540). In addition, natural or artificial flavorings or sweeteners may be added to enhance the taste of topical preparations applied for local effect to mucosal surfaces. Inert dyes or colors may be added, particularly in the case of preparations designed for application to oral mucosal surfaces.

Suitable vehicles or carriers for topical application may contain a daily dose of between 0.1 mg and 120 g of 0.001% to 100% (all percentages are by weight) of the active compound, and may be prepared by conventional techniques to be in conventional forms such as lotions, suspensions, ointments, creams, gels, tinctures, suppositories, elixirs, solutions, aerosols, sprays, powders, pastes or slow-release polymers for topical application; or mouth rinses or rectal or vaginal suppositories for local/topical application to these respective mucosal surfaces. Suitable pharmaceutical diluents or carriers include water, alcohols, sterols, propylene glycol, polyethylene glycol, glycerin, diisopropyl adipate, 1,2,6-hexanetriol, isopropyl myristate, propylene carbonate, natural and/or synthetic or hardened oils and waxes, kaolin, talc, titanium dioxide, as well as suitable solubilizers or emulsifying agents such as ethyl-hydroxybenzoate, cholesterol, sodium laureth sulfate, sodium lauryl sulfate, sorbitan esters, cetyl alcohol, cetearyl alcohol, stearyl alcohol, or stearic acid. Stabilizers such as benzyl alcohol, sodium bisulfite, edetate disodium, citric acid, chlorocresol, butylated hydroxyanisole and butylated hydroxytoluene may be added. Thickening agents such as petrolatum, beeswax, xanthan gum, or polyethylene, plus humectants such as sorbitol solution may also be added. Similarly, emollients such as mineral oil, lanolin and its derivatives, or squalane can be included as part of the topical formulation. Natural or artificial sweeteners including glucose, fructose, sucrose, aspartame, or saccharin may be added to enhance the palatability of preparations applied to mucosal surfaces. Similarly, flavorings such as peppermint oil may be added. Inert dyes such as yellow dye number 6 may be added, particularly in the case of preparations designed for topical application to oral mucosal surfaces.

### Example 1: Suppression of oxazolone-induced contact hypersensitivity by topically-applied reserpine.

One important composition of the subject invention contains about 3.7% by weight of reserpine (reserpine tablets, distributed by Schein Pharmaceuticals, Inc., Port Washington, NY, 11050 and manufactured by Richlyn Labs of Philadelphia, PA, 19124). Reserpine was extracted from the tablets by dissolving the tablets in ethanol, centrifuging the suspension twice and lyophilizing the supernatant. The resultant dried powder was dissolved in about 47.5% ethanol, 10% water, 1% sodium laureth sulfate, 4% isopropyl alcohol, and 37.5% propylene glycol, to make a solution having about 3.7% reserpine by weight.

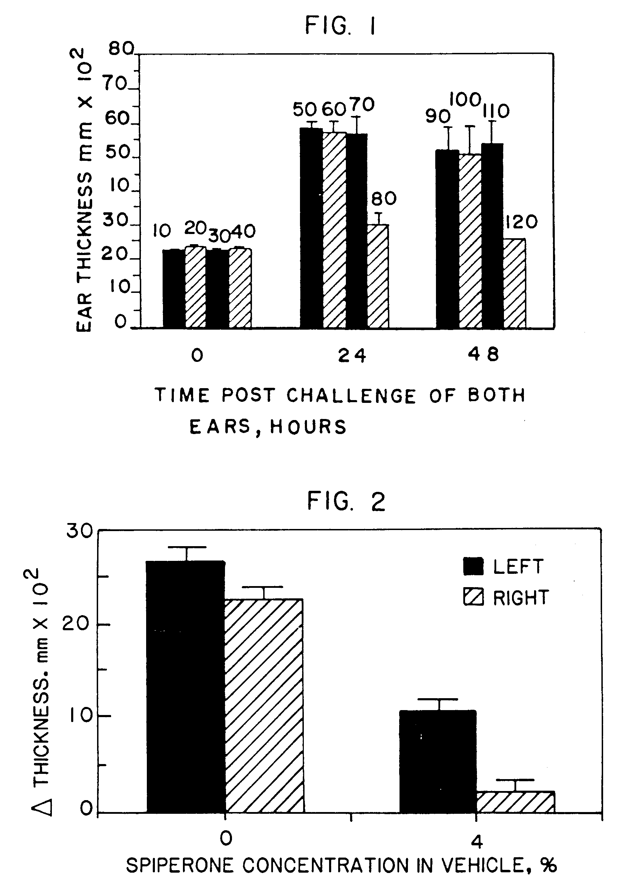

Fig. 1 demonstrates the therapeutic effect of 20µl of the above reserpine preparation on the expression of contact sensitivity reactions in the right ears of mice. Mice (C57BL/6J female mice, 6-8 weeks old: Jackson Laboratory, Bar Harbor, ME; or Balb/c female mice, 6-8 weeks old: Charles River Laboratories, Kingston Facility, Stoneridge, NY) were sensitized to 3% oxazolone (4-ethoxymethylene- 2-phenyl-oxazol-5-one) in 4:1 acetone/olive oil, by applying 50µl to the shaved abdomen and 5µl to each hind footpad of each mouse, seven days earlier. On the day of treatment both the inner and outer surfaces of both ears of each mouse were challenged with 10µl of 0.5% oxazolone in 4:1 acetone/olive oil (i.e. 20µl per ear). One hour after challenging, the control preparation or reserpine preparation was administered by applying 10µl of a given preparation to each side of a treated ear (iNeN 20µl per ear). Ear thicknesses of the mice were measured just before challenging with oxazolone (bars 10, 20, 30, and 40). Of those mice given the reserpine preparation only the right ears were treated (bars 80 and 120). The reserpine preparation reduced the oxazolone-induced inflammation (i.e. the contact sensitivity reaction to oxazolone) significantly in the right ears of treated mice at 24 hours (bar 80) and at 48 hours (bar 120) after challenge with oxazolone, as compared to the right ears of mice treated with 20µl of the vehicle preparation without reserpine (bars 60 and 100). Similarly, the left ears of the mice treated on the right ear with reserpine showed no decrease in swelling (bars 70 and 110); ear thickness measurements of these ears were the same as those of ears treated with the vehicle preparation without reserpine (bars 60 and 100), or those of the corresponding left ears of the mice treated on the right ears with vehicle lacking reserpine (bars 50 and 90). The occurrence of undiminished swelling (increase in ear thickness) in the left ears of those mice treated with reserpine topically on the right ear shows that the effect of topical reserpine is a local, rather than a systemic, effect. Additionally, another vehicle preparation (not containing the active ingredient, reserpine) has been shown to be ineffective in suppressing the ear swelling response in either the treated or the untreated ear. This vehicle was composed of about 1.86% water, 1.06% glycerin, 0.026% peppermint oil, 0.026% saccharin, 17% polyethylene glycol 400 (PEG 400), 40% ethanol, 40% propylene glycol. The lack of suppression of ear swelling by two complex vehicles (lacking the active ingredient reserpine) is evidence that the serotonin antagonist reserpine is needed for this effect.

### Example 2: Suppression of oxazolone-induced contact hypersensitivity by topically-applied spiperone.

A composition of 4% w/w spiperone (1) (Sigma Chemical Co., St. Louis, Mo.) in a vehicle for topical administration (prepared as a suspension of 4 ml absolute ethanol, 1 ml of propylene glycol, and 5 ml of olive oil) was utilized to test the ability of spiperone to suppress the contact hypersensitivity response when applied topically.

The abdomens of C57BL/6J mice were shaved with electric clippers. 50µl of 4% w/w of oxazolone (Sigma Chemical Co.) in a 4:1 v:v acetone:olive oil solution was applied to the shaved abdomen and 5µl was applied to each hind footpad of each mouse. Five to seven days later, mice were challenged for contact hypersensitivity to oxazolone by applying 10µl of the 0.4% oxazolone solution to both the inner and the outer surfaces of each of both ears of each mouse. At one hour after the oxazolone challenge, the right ear of each mouse was topically treated either with 4% spiperone in vehicle or with vehicle alone, by applying 10µl of the test solution to each surface of the ear (20µl total per ear). Before and at 24 hours after the oxazolone challenge, ear thicknesses were measured with a spring-loaded engineer's micrometer. After measurement, mice were sacrificed and specimens of tissue from the right ear were fixed in 10% buffered formalin for at least 48 hours, and then prepared for microscopic assessment of infiltrating cells using standard techniques of morphometry performed on paraffin-embedded and hematoxylin- and eosin-stained sections. Using an ocular grid, specimens were examined in a coded fashion and all morphologically-identified inflammatory cells were quantified.

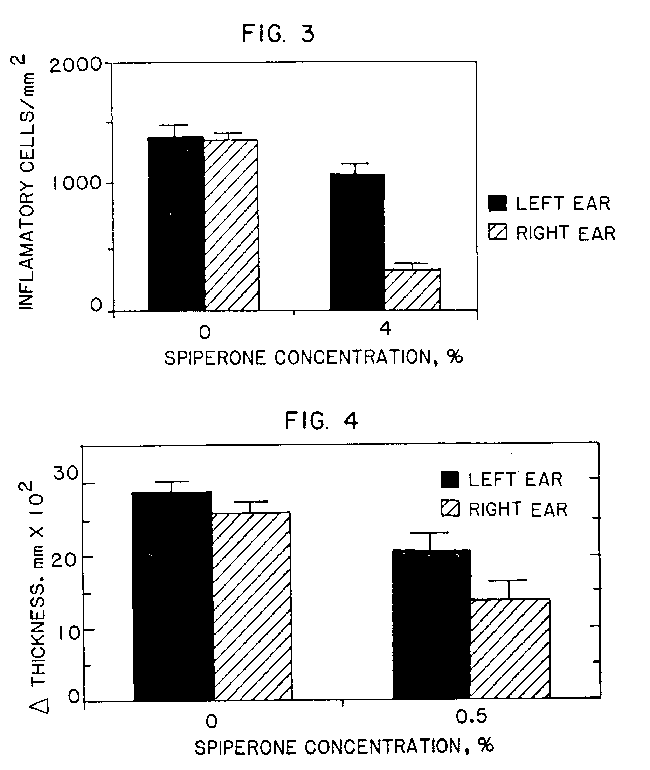

Topical treatment with spiperone reduces the extent of oxazolone-induced cutaneous contact hypersensitivity at the site of spiperone treatment, whether such hypersensitivity is expressed as ear swelling ("Δ ear thickness") (Fig. 2), or by the degree of infiltration of inflammatory cells (Fig. 3). The Δ ear thickness after topical treatment with 4% spiperone was approximately 90% lower than the Δ ear thickness seen with the untreated control mice (Fig. 2), while the number of inflammatory cells per mm² of biopsied ear tissue decreased by approximately 76% compared to the untreated control mice (Fig. 3). Topical treatment of the right ear with spiperone also reduced ear swelling and leukocyte infiltration in the left (untreated) oxazolone-challenged ear (reductions of 60% and 22%, respectively, compared to control mice not treated with spiperone on either ear), indicating that spiperone, at that dose level, can exert a systemic effect when applied to a skin surface.

### Example 3: Suppression of DNFB-induced contact hypersensitivity by topically-applied spiperone.

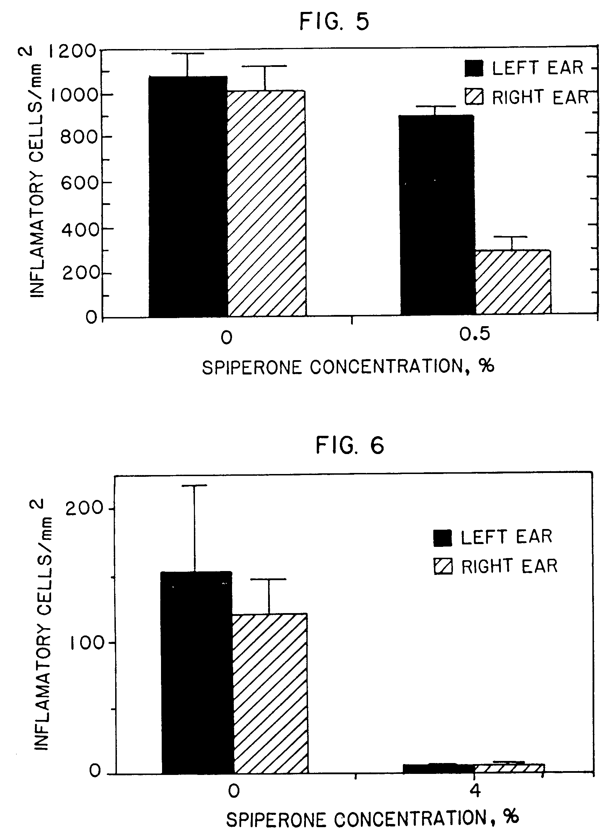

The procedure described in Example 2 was repeated, substituting (a) 0.2% 2,4-dinitro-1-fluorobenzene (dinitrofluorobenzene, DNFB) in acetone (v/v) for both sensitization and elicitation of contact hypersensitivity for the oxazolone solution of Example 2, and (b) a 0.5% (w/w) solution of spiperone (1) in absolute ethanol for the 4% solution of Example 2. As shown in Fig. 4, treatment of the right ear with 0.5% spiperone reduced swelling in that ear by 46% and in the left (untreated) ear by 28%, compared to the ears of control mice which received no spiperone treatment. Fig. 5 illustrates the reduction in numbers of infiltrating inflammatory cells in mice treated with 0.5% spiperone on the right ear. Such cell counts were reduced by 71% in the right (treated) ear and 18% in the left (untreated) ear, compared to the counts in the ears of untreated control mice.

### Example 4: Suppression of rIL-1 induced inflammation by topically-applied spiperone.

0.05 ml of phosphate-buffered saline ("PBS"; GIBCO, Grand Island, NY) containing 200 units of recombinant human interleukin-1 ("rIL-1"; Genzyme Corporation, Cambridge, MA; specific activity: 100,000 units/µg) was injected intradermally into both ears of each of 12 Balb/c mice. At one hour after injection of the rIL-1, the right ear of each mouse was topically treated either with 4% spiperone in vehicle (absolute ethanol), or with vehicle alone, as in Example 2, by applying 10µl of the test solution to each surface of the ear (20µl total per ear). At eight hours after rIL-1 injection, ear thicknesses were measured with a spring-loaded engineer's micrometer, the mice were sacrificed, and ear tissue was processed for microscopic assessment of infiltrating inflammatory cells as described in Example 2.

As shown in Fig. 6, treatment with 4% spiperone is capable of inhibiting rIL-1-induced inflammatory cell infiltration by over 90% compared to the control mice who received no spiperone, whether such inflammatory cell counts were measured in the right ear, which received the topical spiperone application, or in the left ear, which was not directly treated with spiperone, but rather reflects the systemic activity of spiperone applied to the right ear (or possibly the spread of the drug to the left ear during grooming by the mice).

### Other Embodiments

Topical preparations of one or more of reserpine, ketanserin, cyproheptadine, spiperone, methysergide, LY 53857, ritanserin, ICI 169-369, risperidone, pipamperone, trazodone, cinanserine, mianserin, LY 281067, and analogues and derivatives thereof, may be used in combination with other active compounds in order to enhance the topical preparation's anti-proliferative, anti-inflammatory, anti-hypersensitivity, or anti-scarring properties, or to achieve additional therapeutic effects such as relief of pain or itching. For example, a topical reserpine preparation, or a preparation including one or more of the other serotonin antagonists, may be combined with one or more anti-fungal agents, anti-bacterial agents, or compounds capable of inhibiting cutaneous leukocyte accumulation, such as topical corticosteroids and calcium channel blockers including nifedipine, verapamil, diltiazam, isradipine, McN-6186 bepridil, niludipine, perhexiline, nicardipine, flunarizine, nilvadipine, nisoldipine, nitrendipine, felodipine cinnarazine, and nimodipine. In order to treat acne, a traditional acne drug such as erythromycin, clindamycin, benzoyl peroxide, or a retinoid could be included as part of the preparation. Another example would be the addition of an antifungal drug such as ketoconazole, itraconazole, clotrimazole, oxiconazole, sulconazole, econazole, other imidazoles, naftifine, ciclopirox olamine, or nystatin, for the treatment of dermatophyte or candida infection. For treatment of conditions associated with hair loss, minoxidil or other agents that promote hair growth could be included as part of the preparation.

In practice, a therapeutic daily dose of a preparation of reserpine or other serotonin antagonist is applied directly to the inflamed area of the skin, eye, or mucosal membrane, and in a short period of time the inflammation is decreased.

## Claims

1. The use of a compound having the structure 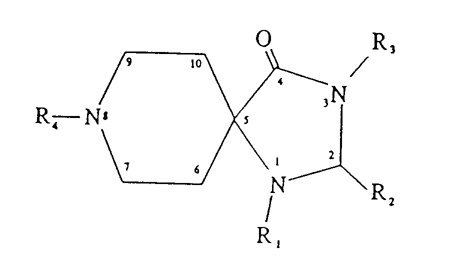 wherein
R₁ = H, CH₃-, C₆H₅-, cyclohexyl, 4-(OCH₃)C₆H₄-, 3-(CH₃)C₆H₄-, 4-(CH₃)C₆H₄-, 4-X-C₆H₄-, (CH₃)₂CH-, CH₃(CH₂)₃-, (CH₃)₂CHCH₂-, CH₃CH₂CH(CH₃)-, or (CH₃)₃C-;
R₂ = H or CH₃;
R₃ = H, CH₃, CH₃CH₂-, CH₃CH₂CH₂-, (CH₃)₂CH-, or CN(CH₂)₂-;
R₄ = H, C₆H₅CH(CH₂CH₃)CH₂-, C₆H₅CH(CH₃)(CH₂)₂-, C₆H₅CH₂CH(CH₃)CH₂-, C₆H₅CH₂CH₂CH(CH₃)-, C₆H₅CH(CH₃)(CH₂)₃-, 4-CH₃C₆H₄CH(CH₃)(CH₂)₃-, 4-(CH₃O)C₆H₄CH(CH₃)(CH₂)₃, 4-X-C₆H₄CH(CH₃)CH₂-, 4-X-C₆H₄CH(CH₂CH₃)CH₂-, 4-X-C₆H₄CH(CH₃)(CH₂)₂-, 4-X-C₆H₄-CH(CH₃)(CH₂)₃-, C₆H₅CH(OCH₃)(CH₂)₂-, 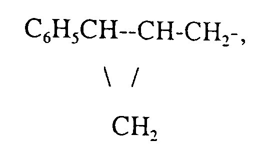 C₆H₅CO(CH₂)₃-, C₆H₅CO(CH₂)₄-, 4-(CH₃)C₆H₄CO(CH₂)₃-, 4-(CH₃O)C₆H₄CO(CH₂)₃-, 4-X-C₆H₄CO(CH₂)₃-, 4-X-C₆H₄CO(CH₂)₃-, 2-thienyl-CO(CH₂)₃-, or 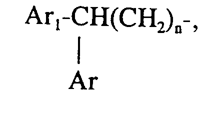 wherein n = 3 or 4; X = F, Cl, or Br; and each of Ar and Ar₁ is,
independently, H, C₆H₅-, 4-(CH₃)C₆H₄-, 4-(CH₃O)C₆H₄-, 4-X-C₆H₄-, 3-(CX₃)C₆H₄-, 2-thienyl, or 4-X-C₆H₄CH₂-in the manufacture of a medicament for immunosuppression.

2. The use according to claim 1 for ocular immunosuppression, for the treatment of ocular inflammation, or for prevention or reduction of the formation of scar tissue in and around the eye.

3. The use according to claim 1 or 2 which is for the treatment of dermatitis, atopic dermatitis, atopic dermatitis, excematous dermatitis, drug eruptions, arthropod bite reactions, inflammatory acne, alopecia areata, male or female pattern alopecia, lichen planus, pyoderma gangrenosa, cutaneous lupus erythematosus, scleroderma, mycosis fungoides, psoriasis, icthyosis, burns, aphthous ulcer, vaginitis, or proctitis.

4. The use according to any one of the preceding claims wherein the compound has the formula

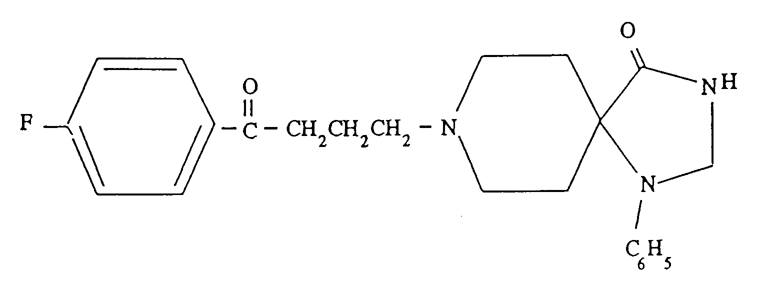

5. The use of any one of the preceding claims, wherein the medicament further comprises one or more additional components capable of inhibiting cutaneous leukocyte accumulation, said additional component(s) being selected from corticosteroids; calcium channel blockers including nifedipine, verapamil, diltiazam, isradipine, McN-6186, bepridil, niludipine, perhexiline, nicardipine, flunarizine, nilvadipine, nisoldipine, nitrendipine, felodipine, cinnarazine, and nimodipine; and other serotonin antagonists such as ketanserin, cyproheptadine, spiperone, methysergide, LY 53857, ritanserin, ICI 169-369, risperidone, pipamperone, trazodone, cinanserine, mianserin, LY 281067, and analogues and derivatives thereof.

6. The use of any one of the preceding claims, wherein said medicament further comprises an additonal component which is active against a fungal infection, said additional component being selected from ketoconazole, itraconazole, clotrimazole, oxiconazole, sulconazole, econazole, other imidazoles, naftifine, ciclopirox olamine, and nystatin.

## Patentansprüche

1. Verwendung einer Verbindung der Formel: 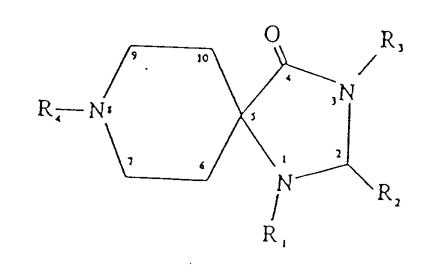 worin bedeuten:
R₁ H, CH₃-, C₆H₅-, Cyclohexyl, 4-(OCH₃)C₆H₄-, 3-(CH₃)C₆H₄-, 4-(CH₃)C₆H₄-, 4-X-C₆H₄-, (CH₃)₂CH-, CH₃(CH₂)₃-, (CH₃)₂CHCH₂-, CH₃CH₂CH(CH₃)- oder (CH₃)₃C-;
R₂ H oder CH₃;
R₃ H, CH₃, CH₃CH₂-, CH₃CH₂CH₂-, (CH₃)₂CH- oder CN(CH₂)₂-;
R₄ H, C₆H₅CH(CH₂CH₃)CH₂-, C₆H₅CH(CH₃)(CH₂)₂-, C₆H₅CH₂CH(CH₃)CH₂-, C₆H₅CH₂CH₂CH(CH₃)-, C₆H₅CH(CH₃)(CH₂)₃-, 4-CH₃C₆H₄CH(CH₃)(CH₂)₃-, 4-(CH₃O)C₆H₄CH(CH₃)(CH₂)₃, 4-X-C₆H₄CH(CH₃)CH₂-, 4-X-C₆H₄CH(CH₂CH₃)CH₃-, 4-X-C₆H₄CH(CH₃)(CH₂)₂-, 4X-C₆H₄-CH(CH₃)(CH₂)₃-, C₆H₅CH(OCH₃)(CH₂)₂-, 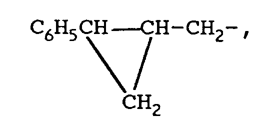 C₆H₅CO(CH₂)₃-, C₆H₅CO(CH₂)₄-, 4-(CH₃)C₆H₄CO(CH₂)₃-, 4-(CH₃O)C₆H₄CO(CH₂)₃-, 4X-C₆H₄CO(CH₂)₃-, 4-X-C₆H₄CO(CH₂)₃-, 2-Thienyl-CO(CH₂)₃- oder 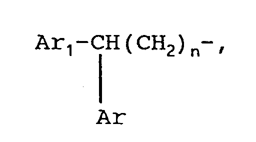 mit n = 3 oder 4; X gleich F, Cl oder Br und Ar und Ar₁ unabhängig voneinander jeweils gleich H, C₆H₅-, 4-(CH₃)C₆H₄-, 4-(CH₃O)C₆H₄-, 4-X-C₆H₄-, 3-(CX₃)C₆H₄-, 2-Thienyl oder 4-X-C₆H₄CH₂-
zur Herstellung eines Medikaments zur Immununterdrückung.

2. Verwendung nach Anspruch 1 zur Okularimmununterdrückung, zur Behandlung von Augenentzündung oder zur Verhinderung oder Verminderung der Bildung von Narbengewebe im und um das Auge.

3. Verwendung nach Anspruch 1 oder 2 zur Behandlung von Dermatitis, atopischer Dermatitis, atopischer Dermatitis, exzementöser Dermatitis, Arzneimittelausschlägen, Reaktionen auf Gliederfüßlerbisse, entzündlicher Akne, Alopecia areata, männlicher oder weiblicher Musteralopecie, Lichen planus, gangrenösem Eiterausschlag, Schmetterlingsflechte der Haut, Sklerodermie, Wucherflechte, Psoriasis, Fischschuppenkrankheit (Ichthyosis), Verbrennungen, aphthösem Geschwur, Vaginitis oder Mastdarmentzündung.

4. Verwendung nach einem der vorhergehenden Ansprüche, wobei die Verbindung der Formel 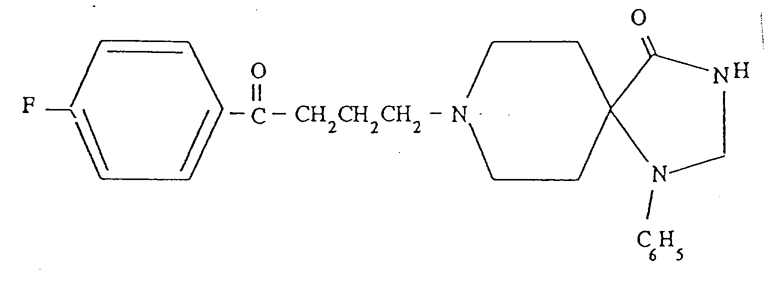 entspricht.

5. Verwendung nach einem der vorhergehenden Ansprüche, wobei das Medikament eine oder mehrere zusätzliche Komponente(n) mit der Fähigkeit zur Hemmung einer Haut-Leukozytenansammlung enthält, wobei die zusätzliche Komponente(n) aus Corticosteroiden, Calciumkanalblockern, einschließlich Nifedipine, Verapamil, Diltiazam, Isradipine, McN-6186, Bepridil, Niludipine, Perhexiline, Nicardipine, Flunarizine, Nilvadipine, Nisoldipine, Nitrendipine, Felodipine, Cinnarazine und Nimodipine, und sonstigen Serotoninantagonisten, wie Ketanserin, Cyproheptadine, Spiperone, Methysergide, LY 53857, Ritanserin, ICI 169-369, Risperidone, Pipamperone, Trazodone, Cinanserine, Mianserin, LY 281067 und Analogen und Derivaten hiervon ausgewählt ist (sind).

6. Verwendung nach einem der vorhergehenden Ansprüche, wobei das Medikament eine weitere gegen Pilzinfektion aktive Komponente enthält, wobei die zusätzliche Komponente aus Ketoconazole, Itraconazole, Clotrimazole, Oxiconazole, Sulconazole, Econazole, anderen Imidazolen, Naftifine, Ciclopiroxolamine und Nystatin ausgewählt ist.

## Revendications

1. Utilisation d'un composé de structure : 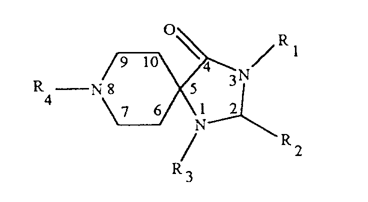 dans laquelle :
R₁ est H, CH₃-, C₆H₅-, cyclohexyle, 4-(OCH₃)C₆H₄-, 3-(CH₃)C₆H₄-, 4-(CH₃)C₆H₄-, 4-X-C₆H₄-, (CH₃)₂CH-, CH₂(CH₂)₃-, (CH₃)₂CHCH₂-, CH₃CH₂CH(CH₃)-, ou (CH₃)₃C- ;
R₂ est H ou CH₃- ;
R₃ est H, CH₃-, CH₃CH₂-, CH₃CH₂CH₂-, (CH₃)₂CH- ou CN-(CH₂)₂- ;
R₄ est H, C₆H₅CH(CH₂CH₃)CH₂-, C₆H₅CH(CH₃)(CH₂)₂-, C₆H₅CH₂CH(CH₃)CH₂-, C₆H₅CH₂CH₂CH(CH₃)-, C₆H₅CH(CH₃)(CH₂)₃-, 4-CH₃C₆H₄CH(CH₃)(CH₂)₃-, 4-(CH₃O)C₆H₄CH(CH₃)(CH₂)₃, 4-X-C₆H₄CH(CH₃)CH₂-, 4-X-C₆H₄CH-(CH₂CH₃)CH₂-, 4-X-C₆H₄CH(CH₃)(CH₂)₂-, 4-X-C₆H₄-CH(CH₃)(CH₂)₃-, C₆H₅CH(OCH₃)(CH₂)₂-, 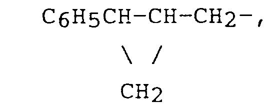 C₆H₅CO(CH₂)₃-, C₆H₅CO(CH₂)₄-, 4-(CH₃)C₆H₄CO(CH₂)₃-, 4-(CH₃O)C₆H₄CO(CH₂)₃-, 4-X-C₆H₄CO(CH₂)₃-, 4-X-C₆H₄CO(CH₂)₃-, 2-thiényl-CO(CH₂)₃- ou 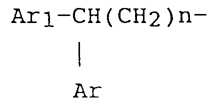 où n vaut 3 ou 4 ; X est F, Cl, ou Br ; et chacun de Ar et Ar₁ est indépendamment H, C₆H₅-, 4-(CH₃)C₆H₄-, 4-(CH₃O)C₆H₄-, 4-X-C₆H₄-, 3-(CX₃)C₆H₄-, 2-thiényle ou 4-X-C₆H₄CH₂-,
pour la fabrication d'un médicament immunosuppresseur.

2. Utilisation selon la revendication 1 destinée à l'immunosuppression oculaire, au traitement de l'inflammation oculaire ou à la prévention ou la réduction de la formation de tissus cicatriciels dans et autour de l'oeil.

3. Utilisation selon la revendication 1 ou 2, destinée au traitement de dermatites, de dermatites atopiques, de dermatites eczémateuses, de dermatites médicamenteuses, de réactions aux morsures d'arthropodes, d'acné inflammatoire, de pelades, de calvities masculines ou féminines, de lichens plans, de pyoderma gangrenosum, de lupus érythémateux cutanés, de sclérodermies, de mycoses fongoïdes, de psoriasis, d'ichtyosis, de brûlures, d'ulcères aphteux, de vaginites ou de proctites.

4. Utilisation selon l'une quelconque des revendications précédentes, dans laquelle le composé est représenté par la formule :

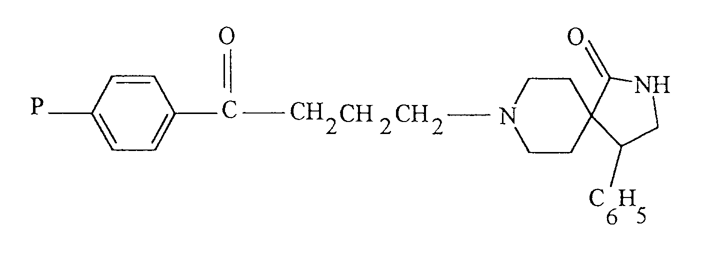

5. Utilisation selon l'une quelconque des revendications précédentes, dans laquelle le médicament comprend en outre un ou plusieurs composant(s) additionnels capables d'inhiber une accumulation leucocytaire cutanée, ledit ou lesdits composants étant choisis parmi les corticostéroïdes ; les inhibiteurs des canaux calciques, dont la nifédipine, le vérapamil, le diltiazam, l'isradipine, le McN-6186, le bépridil, la niludipine, la perhexiline, la nicardipine, la flunarizine, la nivaldipine, la nisoldipine, la nitrendipine, la félodipine, la cinnarazine et la nimodipine ; et d'autres antagonistes de la sérotonine tels que la kétansérine, la cyproheptadine, la spipérone, le méthysergide, le LY 53857, la ritansérine, le ICI 169-369, la rispéridone, la pipampérone, le trazodone, la cinansérine, la miansérine, le LY 281067, ainsi que leurs produits analogues et dérivés.

6. Utilisation selon l'une quelconque des revendications précédentes, dans laquelle ledit médicament comprend en outre un composant additionnel qui est actif vis-à-vis d'une infection fongique, ledit composant additionnel étant choisi parmi le kétoconazole, l'itraconazole, le clotrimazole, l'oxiconazole, le sulconazole, l'éconazole, d'autres imidazoles, la naftiline, le ciclopirox, l'olamine et la nystatine.
